# EUROPEAN PATENT SPECIFICATION

(11) **EP 2 704 685 B1**
(45) Date of publication and mention of the grant of the patent: **29.03.2017**
(21) Application number: 12719362.1
(22) Date of filing: 02.05.2012
(51) Int. Cl.: A61K 8/73, A61K 8/81, A61Q 5/02, A61Q 5/12

(54) **HAIR CARE COMPOSITION COMPRISING HYDROPHOBICALLY-MODIFIED ANIONIC POLYMER AND A METHYLCELLULOSE**
HAARPFLEGEZUSAMMENSETZUNG MIT HYDROPHOB MODIFIZIERTEM ANIONISCHEM POLYMER UND EINER METHYLCELLULOSE
COMPOSITION DE SOIN POUR LES CHEVEUX COMPRENANT UN POLYMÈRE ANIONIQUE À MODIFICATION HYDROPHOBE ET UNE MÉTHYLCELLULOSE

(30) Priority: 04.05.2011 WO PCT/CN2011/073644
(43) Date of publication of application: 12.03.2014
(73) Proprietor: Unilever PLC, London, Greater London EC4Y 0DY (GB); Unilever N.V., 3013 AL Rotterdam (NL)
(72) Inventor: CHUCHOTIROS, Apirudee, Lardkrabang Bangkok 10520 (TH); GILES, Colin, Christopher, David, Bebington Wirral Merseyside CH63 3JW (GB); ZHENG, Bin, Shanghai 200335 (CN)
(74) Representative: Chisem, Janet
(86) International application number: PCT/EP2012/058025
(87) International publication number: WO 2012/150260

(56) References cited:
- WO-A1-2007/088179
- WO-A1-2009/153281
- WO-A2-2011/131452
- GB-A- 688 465
- US-A- 5 346 642
- US-A- 5 827 920
- US-A1- 2007 292 359
- "Foam Enhancement Thickening Gelling Film Formation Stabilization METHOCEL cellulose ethers", amerchol , August 2005 (2005-08), XP002682963, Retrieved from the Internet: URL:http://www.dow.com/PublishedLiterature /dh_0050/0901b80380050865.pdf?filepath=ame rchol/pdfs/noreg/324-00180.pdf&fromPage=Ge tDoc [retrieved on 2012-09-05]

## Description

The present invention relates to a shampoo composition with improved aesthetics.

US 2007/292359 A1 discloses a foamable pharmaceutical carrier comprising polypropylene glycol (PPG) alkyl ether, a surface-active agent water and a liquefied hydrocarbon gas propellant; and pharmaceutical compositions thereof.

WO 2007/088179 A1discloses a gel-like cosmetic preparation for creating hairstyles, comprising a thickening polymer, preferably an anionic, one or more film-forming polymer, preferably a VPNA copolymer, a conditioning polymer, preferably a cationic cellulose, a high molecular weight polyethylene glycol, preferably with a molar mass of from 3 000 000 to 5 000 000 g/mol, preferably 3 500 000 to 4 500 000 g/mol, water, characterized in that the filament break-up time of the preparation, measured using a capillary break-up extensional Rheometer (CaBERTM, Thermo Haake), is between 1 s and 5.0 s, preferably between 1.5 s and 4.0, particularly preferably between 2.0 and 3.5.

GB 688 465 A discloses a hair shampoo liquid comprises an emulsion containing a synthetic detergent consisting of one or more water-soluble salts of sulphated long-chain alkyl alcohols containing at least 8 carbon atoms, minor proportions of vegetable oil and wax and sufficient water to achieve the desired consistency.

"Foam Enhancement Thickening Gelling Film Formation Stabilization METHOCEL cellulose ethers", Amerchol, August 2005,(URL: http://www.dow.com/webapps/lit/litorder.asp?filepath=amerchol/pdfs/noreg/324-00180.pdf&pdf=true) discloses that METHOCEL* cellulose ethers are an extensive family of methylcellulose (MC) and hydroxypropylmethylcellulose (HPMC) polymers that are used globally in many personal care products. They are primarily used as polymeric surfactants, thickeners in liquid formulations, and as binders in solid and semi-solid products.

US 5 346 642 A discloses a fiber conditioning composition, which is preferably a shampoo for human hair, in stable and pearlescent liquid or semi-liquid or gel condition, is an emulsion or dispersion which includes a surfactant, a water insoluble fiber conditioning agent, a long chain saturated primary alcohol or derivative thereof, which stabilizes the emulsion or suspension and makes it pearlescent, and an aqueous medium, which may also contain other components of such fiber conditioning and shampoo compositions.

WO 2009/153281 A1 discloses a hair conditioning composition comprising hydrophobically modified anionic polymer.

Despite the prior art there remains a need for improved shampoo compositions.

Accordingly, the present invention provides a shampoo composition comprising C6-30 alkyl-modified (meth)acrylate polymer,a methylcellulose, and a silicone. The C6-30 alky-modified (meth)acrylate provides better rinse-off properties, the alkyl group comprises from 6 to 30 carbons, more preferably from 16 to 28 and most preferably from 18 to 24 carbons.

A preferred polymer is sold by Rohm & Haas under the tradename Aculyn. The most preferred of which is Aculyn 28™.

The polymer is present at from 0.01 to 5% wt. and more preferably from 0.05 to 1% wt.

Preferably, the composition comprises from 0.01 to 3%, more preferably 0.01 to 2%, even more preferably from 0.05 to 0.5% wt and most preferably from 0.15 to 0.25% wt. of the composition methylcellulose.

Preferably, the composition comprises a suspending agent. The suspending agent useful in the present compositions can be any of several long chain acyl derivative materials or mixtures of such materials. Included are ethylene glycol esters of fatty acids having from 16 to 22 carbon atoms. Preferred are the ethylene glycol stearates, both mono and distearate, but particularly the distearate containing less than about 7% of the mono stearate. Other suspending agents found useful are alkanol amides of fatty acids, having from 16 to 22 carbon atoms, preferably about 16 to 18 carbon atoms. Preferred alkanol amides are stearic monoethanolamide, stearic diethanolamide, stearic monoisopropanolamide and stearic monoethanolamide stearate.

Still other suitable suspending agents are alkyl (C₁₆₋₂₂) dimethyl amine oxides such as stearyl dimethyl amine oxide.

The suspending agent is present at a level of from 0.50% to 5.0%, preferably from 0.5% to 3.0% and most preferably from 0.6 to 0.9% wt. of the composition. Mixtures of suspending agents are also suitable for use in the compositions of this invention.

Preferably, the composition also comprises a pearlescer system comprising a mica component or a bismuth oxychloride component.

Where the pearlescer system comprises a bismuth oxychloride component it is preferred that the composition comprises from 0.01 to 5% wt. bismuth oxychloride component, more preferably, from 0.1 to 0.5% wt of the composition.

Preferably, the bismuth oxychloride component is present with ethylhexyl hydroxystearate. More preferably, the bismuth oxychloride component comprises from 50 to 100% wt. bismuth oxychloride and from 0 to 50% wt. ethylhexyl hydroxystearate.

Preferably, the composition comprises from 0.01 to 5% wt. mica component, more preferably, from 0.1 to 0.5% wt of the composition.

Preferably, the mica component is titanium dioxide coated mica and comprises from 0.01 to 30% wt. titanium dioxide.

The composition is a shampoo composition. Preferably, the composition comprises a cleansing surfactant selected from anionic, non-ionic and amphoteric surfactants.

Preferably, the cleaning phase comprises an anionic surfactant. The anionic surfactant is a salt and has from 8 to 14 carbons, more preferably from 10 to 12 and most preferably 12 carbons. More preferably, these carbons are present in a single alkyl group.

Preferably, the salt is a sulphate, sulphonate, sarcosinate or isethionate.

Preferably, the anionic surfactant is selected from ammonium lauryl sulphate, ammonium laureth sulphate, trimethylamine lauryl sulphate, trimethylamine laureth sulphate, triethanolamine lauryl sulphate, trimethylethanolamine laureth sulphate, monoethanolamine lauryl sulphate, monoethanolamine laureth sulphate, diethanolamine lauryl sulphate, diethanolamine laureth sulphate, lauric monoglyceride sodium sulphate, sodium lauryl sulphate, sodium laureth sulphate, potassium lauryl sulphate, potassium laureth sulphate, sodium lauryl sarcosinate, sodium lauroyl sarcosinate, lauryl sarcosine, ammonium cocoyl sulphate, ammonium lauroyl sulphate, sodium cocoyl sulphate, sodium lauryl sulphate, potassium cocoyl sulphate, potassium lauryl sulphate, monoethanolamine cocoyl sulphate, monoethanolamine lauryl sulphate, sodium tridecyl benzene sulphonate, sodium dodecyl benzene sulphonate, sodium cocoyl isethionate and mixtures thereof.

Preferred anionic surfactants include alkali metal alkyl sulphates, more preferably the alkyl ether sulphates. Particularly preferred anionic cleansing surfactants include sodium lauryl ether sulphate.

The invention encompasses both regular cleansing compositions comprising typical levels of cleansing surfactant as well as concentrated compositions. In a regular composition the level of cleansing surfactant is preferably from 5 to 26% by weight of the total composition while for concentrated compositions the level of cleansing surfactant is from 27 to 70% by weight of the total composition.

Preferably the surfactant is anionic, preferably the composition comprises 5 to 50% anionic surfactant.

The composition according to the invention preferably comprises a cationic deposition polymer.

Suitable cationic deposition aid polymers may be homopolymers which are cationically substituted or may be formed from two or more types of monomers. The weight average (M_{w}) molecular weight of the polymers will generally be between 100 000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15 and JAGUAR C17.

The cationic deposition polymer may be a hydrophobically modified cationic deposition polymer having a carbon chain having from 14 to 30 carbons. It is preferred if the carbon chain is a single alkyl chain, more preferably unbranched.

The hydrophobic modified cationic polymers can comprise mixtures of monomer units derived from amine- and/or quaternary ammonium-substituted monomer and/or compatible spacer monomers.

The hydrophobically cationic deposition polymer can be obtained from hydrophobically modifying deposition polymers from the group consisting of guar, locust bean, tara gum, honey locust, cassia, fenugreek and flame tree. Others useful polymers could include xanthan gum, gellan gum, welan gum, rhamsan gum, konjac, mannan, gum Arabic, soy polysaccharide, xylofructose gums, polyglucose (starch) and tamarind gum.

Mixtures of any of the above cationic polymers may be used.

Cationic polymer will generally be present in a shampoo composition of the invention at levels of from 0.01 to 5%, preferably from 0.05 to 2%, more preferably from 0.07 to 1.2% by total weight of cationic polymer based on the total weight of the composition.

Preferably, the composition comprises an active material. A preferred active material is a fragrance. The fragrance may be solid or liquid. It may and be a single fragrant compound, a natural scented oil, or a mixture of fragrant compounds and/or natural oils. The fragrance can alternatively comprise a chemically protected fragrance compound such as a reaction product of the fragrance compound.

An alternative type of active material which can be incorporated in the controlled release composition is a sunscreen. Examples of sunscreens include those which absorb ultraviolet light between about 290-320 nanometers (the UV-B region) such as para-aminobenzoic acid derivatives and cinnamates such as octyl methoxycinnamate or 2-ethoxyethyl p-methoxycinnamate; and those which absorb ultraviolet light in the range of 320-400 nanometers (the UV-A region) such is benzophenones and butyl methoxy dibenzoylmethane. Additional examples of sunscreen chemicals which may be used as active material in the present invention include menthyl anthranilate; homomenthyl salicylate; glyceryl p-aminobenzoate; isobutyl p-aminobenzoate; isoamyl p-dimethylaminobenzoate; 2,2'-dihydroxy-4-methoxybenzophenone; 2-hydroxy-4-methoxybenzophenone; 4-mono and 4-bis(3-hydroxy-propyl)amino isomers of ethyl benzoate; and 2-ethylhexyl p- dimethylaminobenzoate. The invention is particularly applicable to lipophilic screening agents, including the family of screening agents derived from dibenzoylmethane and more especially 4-tert-butyl-4'-methoxydibenzoylmethane, which effectively have a high intrinsic power of absorption. These dibenzoylmethane derivatives are well known as UV-A active screening agents and are described in particular in European patent application EP-A- 0,114,607. 4-(tert-butyl)-4'-methoxydibenzoylmethane is sold under the trade mark "Parsol 1789" by Givaudan. Another dibenzoylmethane derivative which is preferred according to the present invention is 4-isopropyldibenzoylmethane, sold under the name "Eusolex 8020" by Merck. Octocrylene, a liquid lipophilic screening agent known for its activity in the UV-B range and sold under the trade mark "Uvinul N 539" by BASF. Another lipophilic (or liposoluble) screening agent which can be used in the invention is p- methylbenzylidenecamphor, which is known as a UV-B absorber and is sold under the trade name "Eusolex 6300" by Merck. The sunscreen can alternatively be a hydrophilic screening agent, for example one or more of those described in Application EP-A-678 292, particularly a 3-benzylidine-2-camphorsulphonic derivative such as benzene-1,4-[di(3- methylidenecamphor-10-sulphonic acid)], known under the trade name Mexoryl SX, or a sulphonic derivative of benzophenone or 2-phenylbenzimidazole-5-sulphonic acid, for example that sold under the trade mark "Eusolex 232" by Merck, benzene-1,4-di(benzimidazol-2-yl-5-sulphonic acid) or benzene-1,4-di(benzoxazol-2-yl-5-sulphonic acid).

Further suitable active materials include vitamins. Some vitamins also have beneficial effects when applied topically and for this reason are popular ingredients in various personal care formulations. Vitamins comprise a variety of different organic compounds such as alcohols, acids, sterols, and quinones. They can be classified into two solubility groups: lipid-soluble vitamins and water-soluble vitamins. Lipid-soluble vitamins that have utility in personal care formulations include retinol (vitamin A), ergocalciferol (vitamin D₂), cholecalciferol (vitamin D₃), phytonadione (vitamin K), and tocopherol (vitamin E). Water-soluble vitamins that have utility in personal care formulations include ascorbic acid (vitamin C), thiamin (vitamin Bi) niacin (nicotinic acid), niacinamide (vitamin B₃), riboflavin (vitamin B₂), pantothenic acid (vitamin B₅), biotin, folic acid, pyridoxine (vitamin B₆), and cyanocobalamin (vitamin B₁₂).

Many of the vitamins that are used in personal care compositions are inherently unstable and therefore present difficulties in the preparation of shelf-stable personal care compositions. The instability of the vitamins is usually related to their susceptibility to oxidation. For this reason, vitamins are often converted into various derivatives that are more stable in personal care formulations. These vitamin derivatives offer other advantages in addition to improved stability. Vitamin derivatives can be more amenable to certain kinds of personal care formulations. For example a lipid-soluble vitamin can be derivatized to produce a water-soluble material that is easier to incorporate into a water-based formulation. Retinol and tocopherol are two lipid-soluble vitamins that are particularly useful in skin care compositions and consequently there are many different derivatives of these two vitamins that are used in personal care compositions. Derivatives of retinol include retinyl palmitate (vitamin A palmitate), retinyl acetate (vitamin A acetate), retinyl linoleate (vitamin A linoelate), and retinyl propionate (vitamin A propioniate). Derivatives of tocopherol include tocopheryl acetate (vitamin E acetate), tocopheryl linoleate (vitamin E linoleate), tocopheryl succinate (vitamin E succinate), tocophereth-5, tocophereth-10, tocophereth-12, tocophereth-18, tocophereth-50 (ethoxlyated vitamin E derivatives), PPG-2 tocophereth-5, PPG-5 tocophereth-2, PPG-10 tocophereth-30, PPG-20 tocophereth-50, PPG-30 tocophereth-70, PPG-70 tocophereth-100 (propoxylated and ethoxylated vitamin E derivatives), and sodium tocopheryl phosphate. The invention can be used to give controlled release of these vitamin derivatives. Derivatives of ascorbic acid (Vitamin C) such as ascorbyl palmitate, ascorbyl dipalmitate, ascorbyl glucoside, ascorbyl tetraisopalmitate, and tetrahexadecyl ascorbate can also be used as the active material, as can vitamin derivatives incorporating two different vitamins in the same compound, for example ascorbyl tocopheryl maleate, potassium ascorbyl tocopheryl phosphate or tocopheryl nicotinate.

The active material can also be a cooling agent (a material which gives a cooling sensation to the skin) such as menthol or other cooling agents described in WO96/19119. The cooling agent can be incorporated in a composition to give prolonged release of the cooling agent.

The shampoo composition of the invention comprises silicone. It is preferable if the silicone is present as emulsified particles. Particularly preferred are non-volatile silicones.

The term "non-volatile" as used herein means that the material in question has a vapour pressure under ambient conditions of 0.2mm Hg or less, preferably about 0.1 mm Hg or less.

By "non-alkyl modified silicone" is generally meant an organosiloxane polymer which does not contain any pendant alkyl group having a hydrocarbyl chain length of C₆ or greater extending from at least one of the silicon atoms forming the polymer backbone.

Suitable non-volatile, non-alkyl modified silicones for use in the invention have a viscosity ranging from 350 to 200,000,000 mm²sec⁻¹ at 25°C. Preferably the viscosity is at least 5,000, more preferably at least 10,000 mm²sec⁻¹ at 25°C. Preferably the viscosity does not exceed 20,000,000, more preferably 10,000,000, most preferably 5,000,000 mm²sec⁻¹ at 25°C.

All silicone viscosities mentioned herein are kinematic viscosities unless otherwise specified, and are generally provided by suppliers of silicones, either as measured at 25°C using calibrated capillary glass viscometers under gravity flow conditions, or as deduced from the molecular weight of the material in question.

Preferred non-volatile, non-alkyl modified silicones for use in the invention have a number average molecular weight (Mₙ) ranging from 10,000 to 1,000,000, more preferably from 100,000 to 500,000 dalton.

Suitable non-volatile, non-alkyl modified silicones for use in the shampoo compositions of the invention may be chemically characterised by the general formula (II):

A(R)₂Si-O-[Si(R)₂-O]ₓ-Si(R)₂A (II)

in which each R is independently selected from C₁₋₄ alkyl or aryl, x is an integer from 200 to 8,000 and each A is independently selected from C₁₋₄ alkyl, C₁₋₄ alkoxy, aryl, aryloxy or hydroxyl.

In preferred materials of general formula (II) for use in the invention, all R groups are methyl and both A groups are either methyl or hydroxyl. Such materials have the CTFA designation "dimethicone" and "dimethiconol" respectively. Most preferably, all R groups are methyl and both A groups are hydroxyl.

Also suitable as non-volatile, non-alkyl modified silicones for use in the shampoo compositions of the invention are aminofunctional polydimethylsiloxanes having the CTFA designation "amodimethicone", and the general formula (III):

HO-[Si(CH₃)₂-O-]ₓ-[Si(R)(R¹-NH-R²NH₂)-O-]_{y}-H (III)

in which R is CH₃ or OH, x and y are independent integers of 1 or more and R¹ and R² are each independently an alkylene group having from 2 to 5 carbon atoms.

Also suitable as non-volatile, non-alkyl modified silicones for use in the shampoocompositions of the invention are aminofunctional polydimethylsiloxanes having the CTFA designation "trimethylsilylamodimethicone", and the general formula (IV):

(CH₃)₃Si-O-[Si(CH₃)₂-O-]ₓ-[Si(CH₃)(R¹-NH-R²NH₂)-O-]_{y}-Si(CH₃)₃ (IV)

in which x and y are independent integers of 1 or more and R¹ and R² are each independently an alkylene group having from 2 to 5 carbon atoms.

Mixtures of any of the above described non-volatile, non-alkyl modified silicones may also be used.

The non-volatile, non-alkyl modified silicone is present as emulsified particles in the shampoo composition of the invention.

The emulsified particles of non-volatile, non-alkyl modified silicone may typically have a Sauter mean particle diameter (D_{3,2}) in the composition of the invention ranging from 0.01 to 10, preferably from 0.1 to 5, more preferably from 0.5 to 2.5 micrometres.

Non-volatile, non-alkyl modified silicones for use in compositions of the invention are available as pre-formed silicone emulsions from suppliers of silicones such as those mentioned above. The use of such pre-formed silicone emulsions is preferred for ease of processing and control of silicone particle size. Such pre-formed silicone emulsions will typically additionally comprise a suitable emulsifier, such as an anionic or non-ionic surfactant, and may be prepared by a chemical emulsification process such as emulsion polymerisation, or by mechanical emulsification using a high shear mixer.

Examples of suitable commercially available pre-formed emulsions are Dow Corning® 1784 Emulsion and Dow Corning® 1785 Emulsion. These are both anionic emulsions of dimethiconol.

The total amount of non-volatile, non-alkyl modified silicone in shampoo compositions of the invention generally ranges from 0.1 to 10%, preferably from 0.5 to 5%, more preferably from 1 to 3% by total weight non-volatile, non-alkyl modified silicone based on the total weight of the composition.

In the composition of the invention, the weight ratio of alkyl modified silicone (as defined above) to non-volatile, non-alkyl modified silicone (as defined above) generally ranges from 10:1 to 1:10, preferably from 1:1 to 1:10, more preferably from 1:2 to 1:8.

Preferably, the shampoo compositions of the invention are aqueous, i.e. they have water or an aqueous solution or a lyotropic liquid crystalline phase as their major component.

Suitably, the composition will comprise from 10 to 98%, preferably from 30 to 95% water by weight based on the total weight of the composition.

Optionally, a composition of the invention may contain further ingredients as described below to enhance performance and/or consumer acceptability.

The composition can include co-surfactants, to help impart aesthetic, physical or cleansing properties to the composition.

An example of a co-surfactant is a nonionic surfactant, which can be included in an amount ranging from 0.5 to 10%, preferably from 0.7 to 6% by weight based on the total weight of the composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or di-ethanolamide and coco monoisopropanolamide. A particularly preferred nonionic surfactant is coco monoethanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

A preferred example of a co-surfactant is an amphoteric or zwitterionic surfactant, which can be included in an amount ranging from 0.5 to about 10%, preferably from 1 to 6% by weight based on the total weight of the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.
The total amount of surfactant (including any co-surfactant, and/or any emulsifier) in a shampoo composition of the invention is generally from 1 to 70%, preferably from 2 to 65%, more preferably from 8 to 60% by total weight surfactant based on the total weight of the composition.

Preferably an aqueous shampoo composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition of the invention at levels of from 0.1 to 10%, preferably from 0.5 to 6%, more preferably from 0.9 to 4% by total weight of suspending agent based on the total weight of the composition.

Preferably, the composition has a viscosity of 2000 to 7000 cPs measures at 30°C, measured on a Brookfield Viscometer using spindle RV5 at 20 rpm.

The composition preferably may comprises an oil. The oil may be any oil commonly used in personal care products for example polyolefin oils, ester oils, triglyceride oils, hydrocarbon oils and mixtures thereof. Preferably, the oil is a light oil. Oils, enhance the conditioning benefits found with compositions of the invention.

Preferred oils include those selected from:
- Oils having viscosities from 0.1 to 500 centipoises measures at 30°C.
- Oils with viscosity above 500 centipoises (500-500000 cps) which contains up to 20% of a lower viscosity fraction (less than 500cps).

One type of preferred oil is a polyalphaolefin oil.

Suitable polyalphaolefin oils include those derived from 1-alkalene monomers having from 6 to 16 carbons, preferably from 6 to 12 carbons. Non limiting examples of materials include 1-hexene, 1-octene, 1-decene, 1-dodecene, 1-tetradecene, 1-hexadecene, branched isomers such as 4-methyl-1-pentene and mixtures thereof.

Preferred polyalphaloefins include polydecenes with tradename Puresyn 6 having a number average molecular weight of about 500, Puresyn 100 having a molecular weight of about 3000 and Puresyn 300 having a molecular weight of about 6000 commercially available from Mobil.

Preferably, the polyalphaolefin oil is present at from 0.05 to 10%, particularly from 0.2 to 5%, and especially from 0.5 to 3% by weight of the composition.

Also suitable are triglyceride oils include fats and oils including natural fats and oils such as jojoba, soybean, sunflower seed oil, rice bran, avocado, almond, olive, sesame, castor, coconut, coconut palm oil, sunflower oil, mink oils; cacao fat; beef tallow, lard; hardened oils obtained by hydrogenating the aforementioned oils; and synthetic mono, di- and triglycerides such as myristic acid glyceride and 2-ethylhexanoic acid glyceride.

Preferably, the triglyceride oil if present is at levels from 0.05 to 10%, particularly from 0.2 to 5%, and especially from 0.5 to 3% by weight of the composition.

Highly suitable oils for use with the present invention are hydrocarbon oils. Hydrocarbon oils have at least 12 carbon atoms, and include paraffin oil, polyolefin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Also suitable are polymeric hydrocarbons of C₂₋₆ alkenyl monomers, such as polyisobutylene.

Preferably, the hydrocarbon oil is present at from 0.05 to 10%, particularly from 0.2 to 5%, and especially from 0.5 to 3% by weight of the composition.

Also suitable are ester oils which have at least 10 carbon atoms, and include esters with hydrocarbyl chains derived from fatty acids or alcohols. Typical ester oils are formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Preferably, the ester oil is present at from 0.05 to 10%, particularly from 0.2 to 5%, and especially from 0.5 to 3% by weight of the composition.

Preferably, the composition comprises a cleansing anionic surfactant which comprises an alkyl group with from 10 to 14 carbons.

A further component that may be used in compositions of the invention is a hydrocarbon oil or ester oil. Like silicone oils, these materials may enhance the conditioning benefits found with compositions of the invention.

Suitable hydrocarbon oils have at least 12 carbon atoms, and include paraffin oil, polyolefin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Also suitable are polymeric hydrocarbons of C₂₋₆ alkenyl monomers, such as polyisobutylene.

Suitable ester oils have at least 10 carbon atoms, and include esters with hydrocarbyl chains derived from fatty acids or alcohols. Typical ester oils are formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Mixtures of any of the above described hydrocarbon/ester oils also be used.

The total combined amount of hydrocarbon oil and ester oil in compositions of the invention may suitably range from 0.05 to 10%, particularly from 0.2 to 5%, and especially from 0.5 to 3% by weight of the composition.

A composition of the invention may contain other ingredients for enhancing performance and/or consumer acceptability. Such ingredients include fragrance, dyes and pigments, pH adjusting agents, pearlescers or opacifiers, viscosity modifiers, and preservatives or antimicrobials. Each of these ingredients will be present in an amount effective to accomplish its purpose. Generally these optional ingredients are included individually at a level of up to 5% by weight of the total composition.

### Mode of Use

The compositions of the invention are primarily intended for topical application to the body, preferably the hair and/or scalp of a human subject in rinse-off compositions.

The compositions provided by the invention are preferably shampoo compositions for the treatment of hair (typically after shampooing) and subsequent rinsing.

The invention will be further illustrated by the following, example in which all percentages quoted are by weight based on total weight unless otherwise stated.

### EXAMPLE 1

The stability of several formulation mixes was investigated. Compositions comprising the test mixtures were made and stored at 45C for 3 months. The results are as follows.

| | **Aculyn 28** | **Aculyn 28 + Methocel 40-202** |
|---|---|---|
| **Appearance** | Pearlescence | Pearlescence |
| **Stable?** | No | Yes |

The results show that the inclusion of methocellulose improved the stability of an Aculyn 28-containing shampoo without any reduction in pearlescence.

### EXAMPLE 2

The following is a shampoo composition made by standard processes.

| **Ingredient** | **% wt.** |
|---|---|
| Sodium laureth sulphate (70%) | 17.14 |
| Cocoamidopropyl betaine (30%) | 5.33 |
| Guar hydroxypropyl trimonium chloride | 0.20 |
| Acrylates/Beheneth-25 Methacrylate Copolymer (20%) | 1.00 |
| Hydroxypropyl methylcellulose | 0.20 |
| Ethylene glycol distearate (25%) | 3.20 |
| Dimethiconol (50%) | 4.00 |
| Fragrance | 0.70 |
| DMDM hydantoin (55%) | 0.10 |
| Sodium chiloride | 0.50 |
| Chlorinated water | To 100 |

## Claims

1. Shampoo composition comprising C6-30 alkyl-modified (meth)acrylate polymer,a methylcellulose and a silicone.

2. Composition according to claim 1 wherein the silicone is in the form of emulsified particles.

## Patentansprüche

1. Shampoo-Zusammensetzung, umfassend C6-30-Alkyl-modifiziertes (Meth)acrylat-Polymer, eine Methylcellulose und ein Silikon.

2. Zusammensetzung nach Anspruch 1, wobei das Silikon in Form emulgierter Partikel vorliegt.

## Revendications

1. Composition de shampooing comprenant un polymère de (méth)acrylate modifié par un groupe alkyle en C6-30, une méthylcellulose et un silicone.

2. Composition selon la revendication 1, dans laquelle le silicone est dans la forme de particules émulsionnées.
